# EUROPEAN PATENT APPLICATION

(11) **EP 2 140 772 A1**
(43) Date of publication of application: **06.01.2010**
(21) Application number: 08159625.6
(22) Date of filing: 03.07.2008
(51) Int. Cl.: A23L 1/30, A61K 35/66, C12N 1/06, C12N 1/16

(54) **Temperature-induced delivery of nutrients by micro-organisms in the gastrointestinal tract**

(71) Applicant: Nestec S.A., 1800 Vevey (CH)
(72) Inventor: Gysler, Christof, 1807 Blonay (CH); Niederberger, Peter, 1066 Epalinges (CH); Page, Nicolas, 1010 Lausanne (CH); Schaffer-Lequart, Christelle, 1083 Mezieres (CH)
(74) Representative: Marquardt, Ulf

(57) **Abstract**

The present invention relates in general to the field of health and well-being. In particular, the present invention relates to temperature sensitive micro-organisms and their use as vehicle for the preparation of a composition to deliver compounds, for example nutrients, to specific regions of a body.

## Description

The present invention relates in general to the field of health and well-being. In particular, the present invention relates to temperature sensitive micro-organisms and their use as vehicle for the preparation of a composition to deliver compounds, for example nutrients, to specific regions of a body.

The incorporation of certain compounds in food matrices may be challenging to food industry today, because of specific properties of these compounds or the food preparation itself. For example, these compounds may be process sensitive, may have a poor stability during storage or transportation, may interact with other food components resulting for example in lipid oxidation and/or discoloration, may have a detrimental impact on taste, may have a poor stability in gastric juice, and/or may be poorly available in the gut.

A number of nutrient delivery vehicles have been developed so far, including protein complexes, vegetable oils or gel beads. However, their use is limited to certain applications, due to moisture sensitivity, food matrix incompatibility, process sensitivity, leakage from the carrier and absence or poor delivery in the gastrointestinal tract.

It would be helpful to have a vehicle that allows the safe transport of sensitive compounds, in particular nutrients, to specific locations of the body, while overcoming the problems listed above.

The gastro-intestinal tract is the major organ for taking up nutritional compounds to be utilized by the organism for maintaining its health and well-being. The intestinal tract is colonized by synbiotic or mutualistic micro-organisms, such as *Bacteroides, Clostridium, Fusobacterium, Eubacterium, Escherichia, Ruminococcus, Peptococcus, Pepstreptococcus, Lactobacillus,* and *Bifidobacterium,* one of the major contribution thereof being to perform a variety of beneficial functions such as breaking down and adding in the absorption of food, stimulating cell growth, repressing growth of harmful bacteria, training the immune system, and defending against some diseases.

In the past, probiotic organisms have attracted a great deal of attention in that some strains have been found to exhibit valuable properties to human and animals upon ingestion. Probiotics are considered to be viable microbes (bacteria and yeasts) which promote the individual's health.

Recently, WO03053474 disclosed the use of bacteria, namely *Lactobacilli, Bifidobacteria, Streptococci, Pediococci, Enterococci, Lactococci, Oenococci, Staphylococci, Bacteroides,* for a directed delivery of substances to specific parts of the gut. The invention disclosed the use of the micro-organisms with specific and possibly altered resistance to gastric and/or bile juices to achieve this objective.

However, there is a broad variability of gastric and bile juice composition due to age, health status, interindividual and interspecies variations. In order to deliver compounds to a large spectrum of organisms into various locations of the gut new tools needed to be developed.

The present invention provides the art with a delivery system for compounds, in particular nutrients that will be released in the body after consumption. In addition, the present invention protects process-sensitive compounds during production and storage of the product, it ensures that the compounds do not interact with other food components resulting for example in lipid oxidation and/or discoloration, and it has a positive impact on taste and it is safe to consume, for example with a food product.

The present inventors were surprised to see that they could achieve this objective by a use in accordance with claim 1.

The present invention relates to a temperature sensitive micro-organism that will fully or at least partially lyse when exposed to temperature range of about 32 to 45°C and that can be used as vehicle for the preparation of a composition to deliver compounds, in particular nutrients, to a portion of the body, in particular to the gastrointestinal tract.

In a preferred embodiment of the present invention, a full or an at least partial lysis means that at least 10 %, preferably at least 20%, more preferably at least 30%, most preferably at least 40% of the micro-organisms, ideally at least 95 % of the temperature sensitive micro-organisms lyse after exposure at 37°C for 5 minutes to 24 hours. It is particularly preferred if all micro-organisms lyse after exposure to 37 °C for less than 24 hours.

An at least partial lysis also comprises a condition where the cell wall and or membranes are permeabilized and/or weakened, so that the carried compounds can leave the cell while a fraction of the population may remain viable.

A micro-organism is regarded as temperature sensitive if it at least partially lyses after exposure to temperatures in the range of about 32 to 45°C for at least 5 minutes.

One embodiment of the present invention is the use of a temperature sensitive micro-organism that will at least partially lyse in the temperature range of about 32 to 45°C as vehicle for the preparation of a composition to deliver nutrients to the gastrointestinal tract.

This method will also allow a targeted release of the compound, for example of nutrients, in specific areas of the body, for example in the gastro-intestinal tract, while improving the compounds bioavailability.

Micro-organisms that can be used will at least partially lyse in the temperature range of 32 to 45°C. It is however preferred for food-applications that the micro-organism used is food grade. A material or micro-organism is "food grade" if it is approved for human or animal consumption.

In a particular preferred embodiment of the present invention, the micro-organism is a yeast, for example a low temperature induced (lti) yeast, preferably a food grade yeast selected from the group consisting of hemiascetomycetous yeast species, Ascomycotina or Deuteromycotina. More preferably the yeast is selected from the group consisting of *Debaryomyces, Kluyveromyces, Saccharomyces, Yarrowia, Zygosaccharomyces, Candida, Schizosaccharomyces* and *Rhodotorula,* and most preferably the yeast is *Saccharomyces cerevisiae* (baker's yeast) and in particular, the temperature sensitive *S. cerevisiae* strains CNCM I-4000, CNCM I-4001, CNCM I-4002, CNCM I-4003, CNCM I-4004, CNCM I-4005, CNCM I-4006, CNCM I-4007, and the temperature sensitive *K. marxianus strains* CNCM I-4009, CNCM I-4010, CNCMI-4011.

Yeasts have the advantage that they generally have a positive impact on the taste of a product, are easy to produce and efficiently accumulate compounds provided by their growth medium or synthesised by themselves.

Under certain circumstances it might even be preferred to use low temperature induced (lti) yeasts for the purpose of the present invention. Lti yeasts are described in the literature for example in EP 0487878, EP 0663441 or EP 1148121. Lti yeasts do not multiply at low temperatures, which has the advantage that - for example - at refrigeration temperatures substantially no fermentation occurs.

Alternatively, the micro-organism may also be a bacterium, in particular selected from the group consisting of lactic acid bacteria, in particular *Lactobacilli* and/or *Bifidobacteria* and, more preferably, *Lactobacillus johnsonii, Lactobacillus reuteri, Lactobacillus rhamnosus, Lactobacillus paracasei, Lactobacillus plantarum, Lactobacillus casei, Bifidobacterium bifidum, Bifidobacterium breve, Bifidobacterium longum, Bifidobacterium animais, Bifidobacterium lactis, Bifidobacterium infantis, Bifidobacterium adolescentis, Bifidobacterium pseudocatenulatum, Bifidobacterium catenulatum* or mixtures thereof and most preferably a temperature sensitive bacterium derived from the strains *Lactobacillus johnsonii* NCC 533 (CNCM I-1225), *Lactobacillus paracasei* NCC2461 (CNCM I-2116), *Lactobacillus rhamnosus* NCC 4007 (CGMCC 1.3724), *Bifidobacterium adolescentis* NCC 251 (CNCM I-2168), *Bifidobacterium longum* NCC 490 (CNCM I-2170), NCC 2705 (CNCM I-2618) and NCC 3001 (ATCC BAA-999), *Bifidobacterium lactis* NCC 2818 (CNCM I-3446), *Bifidobacterium breve* NCC 2950 (CNCM I-3865), NCC 466 (CNCM I-3914).

Bacteria have the advantage that they are easy to produce in large quantities and that they are often already present naturally in the gut, so that a body is not confronted with foreign species.

A mixture of different micro-organisms may also be employed.

Such temperature sensitive micro-organisms may be identified for example by natural selection. One possible experimental essay for the identification of temperature sensitive micro-organisms is described in Example 1b. Yeasts, for example, can be rendered temperature sensitive by a mutation in the pkc1 gene.

Alternatively, the micro-organisms may also be made temperature sensitive by other methods. For example, the micro-organisms may be pre-treated. Such a pre-treatment may include weakening the viability of the micro-organisms, so as to reduce their overall growth or endurance performance. For example, an appropriate pre-treatment may be effected by subjecting the micro-organisms to a shock treatment, e. g. exposing them to specific conditions of heat, low temperatures or low or high pH-values, respectively, incubating them with phages or inserting recombinant constructs into the micro-organisms which effect an early lysis of the cells, enzymatic treatment to damage the cell wall, using micro-organisms known to contain prophages, subjecting them to particular fermentative conditions non favourable for growth, triggering an SOS-response or inactivating the intrinsic protective stress- response systems, which repair damaged intracellular proteins on a regulatory or gene-expression level, so that the resulting micro-organisms will eventually exhibit a reduced viability and/or resistance to environmental conditions/changes in the gut.

Furthermore, temperature sensitive mutants can be obtained for example from bacteria or from hemi-ascetomycetous yeast species such as *S. cerevisiae, K. lactis* or *S. pombe* by conventional methods (chemical or radiation induced mutagenesis) and out-crossing of undesired mutations. Desired mutations have a conditional temperature sensitive cell lysis phenotype, which cause the release of incorporated nutrients at temperature above 30°C. 8 such temperature-sensitive mutants (cly1 to cly8) were already described in the literature (Hartwell, L.H. (1967) Macromolecule synthesis in temperature sensitive mutants of yeast J.Bact. 93: 1662-1670).

Suitable temperature sensitive *Saccharomyces cerevisiae* yeast may be selected from mutant strains consisting of the genetic complementation groups cly 1, cly 2, cly 3, cly 4, cly 5, cly 6, cly 7, cly 8 or combinations thereof.

Temperature sensitive are often described to have an alteration in the genes regulating the polarized growth, the septation, and/or the maintenance of the cell wall integrity.

If the yeast is *Saccharomyces cerevisiae,* these genes may be preferably selected from the group consisting of *BCK1, CDC12, CDC15, CDC31, CDC24, GLC7, GPI7, HSF1, IQG1, KIC1, LSTB, MID2, WSC1, MOT2, MPK1, MPTS, SSD1, MSS4, NUD1, PKC1, PMT2, PMT4, PPH22, PPZ1, PPZ2, RH03* and *RHO4, ROM1, ROM2, SRB1, SWI4, SWM1, TIF51A, TSC11, TOR1, TCO89, TAP42, TOR2, BIG1, TUS1, VIG9, WSC1, WSC2, WSC3, YPK1, YPK2, YPK1, YKR2,* the seven yapsins, and combinations thereof.

If the yeast is *Schizosaccharomyces pombe,* the genes are preferably selected from the group consisting of *ags1, bgs3, bgs4, cwg1, orb11, cps1, cwg1, cwg2, ehs1, PP2C, psu1, rgf1, rgf3, rho1, sid1, sid2, sid4, spg1* and *mob1,* and combinations thereof.

One typical example of a temperature sensitive *S. cerevisiae* strain is a *cly5* mutant; the target gene of S. *cerevisiae* is *PKC1* which codes for a protein kinase C. The C to T transition mutation at nucleotide 2871 leads to a Threonine to Isoleucine change at amino acid 958. The altered polypeptide confers temperature sensitivity to the mutant: it can grow and accumulate nutrients (e.g. iron) from the culture medium when growing at 23-25 °C, but at 37°C the yeast cells are unable to replicate and will lyse because the ts mutation interferes with the repairing and growth capacity of the cell wall. Therefore, living nutrient-enriched mutant cells ingested together with appropriate food products will lyse in the body due to the onset of budding and will deliver the nutrient in the body at 37°C.

If an Iti-yeast (low temperature inactive) strain is used and made temperature sensitive, e.g., by one of the methods described above, this will have the advantage that such a Iti-yeast strain will cause no fermentation under refrigerated storage conditions, while only at elevated temperatures, for example room temperature or above the Iti-yeast strain will start to multiply. At more elevated temperatures, for example at temperatures above 35 °C, it will then lyse and release its valuable content.

Compounds that are to be delivered by the micro-organism in accordance with the present invention can be incorporated into the micro-organism by any means known in the art.

There are different means to incorporate nutrients into micro-organsims, in particular into yeast. Yeast can naturally take up and accumulate molecules which are present in their growth environment or synthesize and accumulate the said nutrients relying on their metabolic activities.

The synthesis of a desired substance not accumulated or synthesized by micro-organisms, in particular yeast, under normal cultivations conditions may also be obtained by alterations in their genes, by e. g. subjecting the micro-organisms to a random mutagenesis and selecting those mutants expressing a higher amount of the desired substance. Yet, also recombinant means may be applied, wherein the expression of the endogeneous or exogenous gene is increased by e. g. linking the corresponding gene with a promotor stronger than the endogenous one, or by inserting the gene or genes encoding the substance (s) of interest into the micro-organism on a plasmid or into the chromosome thereof, optionally linked with a strong promoter that drives the expression of the gene (s) of interest such that the recombinant micro-organism will contain higher amounts of the desired substance.

For example, the compounds may be synthesized by the micro-organism itself, preferably as part of the micro-organism natural metabolism.

The micro-organisms may also be modified by biotechnological means, for example, by the introduction of an expression vector that causes the micro-organism to produce a compound of interest that the vector codes for. Similarly, the natural synthesis of compounds by the micro-organism might be enhanced, for example by introduction of stronger promoters.

The micro-organisms may also accumulate the compounds to be delivered from the outside. Such compounds may be present in the growth media of the micro-organism cultures. For example, yeasts naturally accumulate many micronutrients such as minerals and vitamins. This way, for example selenium-enriched yeast may easily be prepared and may be used to supplement a dietary composition to treat or prevent selenium deficiency.

Of course also combinations of these methods to include the compounds of interest into the micro-organisms may be used.

Consequently, in one embodiment of the present invention the compounds, preferably the nutrients, are incorporated into the micro-organism, preferably the yeast, by growing the micro-organism on a medium enriched with these compounds. Alternatively or additionally the micro-organism may synthesize the compounds itself.

The compounds may be any compounds that can be synthesized by the micro-organism, possibly after biotechnological modification, or may be taken up by the micro-organism during growth and/or cultivation.

It is however preferred that the compound is a nutrient.

Nutrients are compounds that are used in organism's metabolism (human, pet, companion animal and livestock). Preferably, nutrients to be delivered are compounds that the organism consuming the cell lysing micro-organism cannot synthesize itself or that the organism can only synthesize in insufficient amounts, so that they must be taken up from the environment. A nutrient is essential to an organism if it cannot be synthesized by the organism in sufficient quantities and must be obtained from an external source. Nutrients needed in relatively large quantities are called macronutrients and those needed in relatively small quantities are called micronutrients.

The micro-organisms of the present invention may be used to deliver both, macronutrients or micronutrients.

Typically, the nutrients may be selected from the group consisting of minerals, preferably iron, selenium, zinc, chromium, copper; vitamins, preferably folic acid, niacin, vitamin C; plant polyphenols, preferably catechins, chlorogenic acids, isoflavones; carotenoids , preferably beta-carotene, astaxanthin, zeaxanthin, lycopen; alkaloids, preferably caffeine; amino acids, preferably cysteine, essential amino acids such as isoleucine, leucine, lysine, threonine, tryptophan, methionine, histidine, valine and phenylalanine; antioxidants; aroma compounds, preferably lemon oil, orange peel oil, thyme, rosemary oil, oregano oil; lipids, preferably polyunsaturated fatty acids, long-chain polyunsaturated fatty acids, fatty amides, glycerophospholipids, glycerides; enzymes and/or co-enzymes, preferably co-enzyme Q10, beta-galactosidase; non secreted heterologous proteins, preferably vaccines or mixtures thereof.

The micro-organisms are conveniently administered in form of a product acceptable to a consumer, such as an ingestible carrier or support, respectively.

The composition prepared by the use of the present invention may be any composition intended for human or animal use.

Preferably the composition is intended for use by humans, pets, companion animals and/or livestock.

The composition may be a food composition, a dietary supplement, a nutraceutical, a drink, an animal food composition, a cosmetic composition, or a medical composition.

Typical cosmetic compositions include for example beauty supplements, such as nutritional beauty supplements, nutricosmetics, oral cosmetics, nutritional supplements, oral supplements and oral skin- and/or hair care products

Typical food compositions may be selected from the group consisting of a cereal product, such as a cereal bar, baby cereals and infants cereals or a chilled dairy product such as a cheese, milk, fermented milk drink, milk based fermented products, curd, ice cream, yoghurt, milk powder and fortified powder drinks such as infant formula and growing-up milks, beverages, sport nutrition and drink composition, soups, culinary products, pet food, tablets, liquid bacterial suspensions, dried oral supplements, wet oral supplements, compositions for dry tube feeding and/or compositions for wet tube feeding.

Such a composition may be prepared by choosing a micro-organism that either already contains the substance (s) to be delivered or that will be modified accordingly in order to provide such (a) substance (s) in a sufficient amount.

Once a micro-organism has been selected and optionally pretreated, said micro-organism may be included in a product as mentioned above in an amount of 10⁴-10¹²cfu/g dry weight of the composition in case of a non-liquid composition and in an amount of 10⁴- 10¹²cfu/ml of the composition in case of a liquid composition, depending on the nature of the substance to be delivered and the amount of the substance contained in the respective micro-organisms.

The loaded micro-organisms may then be incorporated into a food matrix by methods that are known in the art, for example for probiotics. For example, a yoghurt containing the micro-organisms used in the present invention may be prepared by adding iron-enriched yeast biomass to the fermented white base. An infant cereal preparation comprising the micro-organisms used in the present invention can be prepared by co-mixing the dry cereal with dry yeast powder.

The compositions may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials.

They may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, they may contain an organic or inorganic carrier material suitable for oral or enteral administration as well as vitamins, minerals trace elements and other micronutrients in accordance with the recommendations of Government bodies such as the USRDA.

The composition of the present invention may contain a protein source, a fat source and/or a carbohydrate source and may be intended for oral, enteral, parenteral or topical application.

If the composition includes a fat source, the fat source more preferably provides 5% to 40% of the energy of the composition; for example 20% to 30% of the energy. DHA may be added. A suitable fat profile may be obtained using a blend of canola oil, corn oil and high-oleic acid sunflower oil.

A source of carbohydrates may preferably provide between 40% to 80% of the energy of the composition. Any suitable carbohydrate may be used, for example sucrose, lactose, glucose, fructose, corn syrup solids, maltodextrins, and mixtures thereof.

Any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred.

The composition of the present invention may also contain prebiotics.

"Prebiotic" means food substances that promote the growth of probiotics in the intestine. They are not broken down in the stomach and/or upper intestine or absorbed in the GI tract of the person ingesting them, but they are fermented by the gastrointestinal microflora and/or by probiotics (Gibson and Roberfroid (1995) Dietary Modulation of the Human Colonic Microbiota: Introducing the Concept of Prebiotics, J. Nutr. 125: 1401-1412).

"Probiotic" means microbial cell preparations or components of microbial cells with a beneficial effect on the health or well-being of the host. (Salminen S et al. (1999) Probiotics: how should they be defined. Trends Food Sci. Technol. 10: 107-10).

Some of the micro-organisms that may be used in the framework of the present invention qualify as probiotics.

The presence of prebiotics will support the viability of the temperature sensitive micro-organism until they are subjected to temperatures above 32 °C.

The prebiotics that may be used in accordance with the present inventions are not particularly limited and include all food substances that promote the growth of probiotics. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (GOS), isomalto-oligosaccharides, xylo-oligosaccharides (XOS), oligosaccharides of soy, glycosylsucrose (GS), lactosucrose (LS), lactulose (LA), palatinose-oligosaccharides (PAO), malto-oligosaccharides (MOS), gums and/or hydrolysates thereof, pectins and/or hydrolysates thereof.

The micro-organisms and the above products may consequently be utilized for supporting the well-being of individuals and/or the treatment and/or the prophylaxis of diseases.

The composition prepared by the use of the present invention may further be used to improve the stability of nutrients, for example during production and storage.

It may also be used to reduce a potential detrimental impact of the compounds to be delivered, for example of the nutrients, on the taste of the composition.

Having the compounds of interest incorporated within the micro-organisms as described above, will also avoid that the compounds to come into contact with other compounds, for example in the food matrix, and consequently will prevent undesired interactions.

Further, the temperature dependant release of the compounds when using the teaching of the present invention will enable a targeted release of compounds, for example nutrients, preferably in the gut or a specific location in the digestive tract.

Further, the use of the present invention allows it to improve the bioaccessibility and/or bioavailability of the compounds to be delivered.

Those skilled in the art will understand that they can freely combine all features of the present invention described herein, without departing from the scope of the invention as disclosed.

Further advantages and features of the present invention are apparent from the following Examples and Figures.

### Figures:

Figure 1A shows the release of intracellular Adenylate Kinase (AK) by the measurement of relative luminescence units (RLU) at 595nm when cell lysis is triggered at 37°C in *S. cerevisiae* lysis mutants and in wild type baker's yeast (Levure Boulangère Bleue (LBB), ex. Lesaffre, Marcq en Baroeul, France).

Figure 1 B shows the release of intracellular AK by the measurement of relative luminescence units (RLU) at 595nm when cell lysis is triggered at 37°C in *K. marxianus* lysis mutants and in wild type (L46).

Figure 1C shows the release of intracellular AK by the measurement of relative luminescence units (RLU) at 595 nm at 37°C in *S. pombe* lysis mutant (Sp132) and in wild-type control (Sp968).

Figure 2 shows the viable cell titer and iron release measurement in simulated duodenal juice at non permissive temperature (37°C) by the wild type and mutant *Saccharomyces cerevisiae* strains.

Figure 3 shows a schematic diagram of a multicompartmental model of the stomach and the small intestine : (a) Gastric compartment; (b) duodenal compartment; (c) jejunal compartment; (d) ileal compartment; (e) basic unit ; (f ) glass jacket ; (g) flexible wall ; (h) rotary pump; (i) water bath; (j) peristaltic pump; (I, m) pH electrodes ; (n, o) syringe pumps; (p) hollow-fibre device. (From Larrsson et al. (1997) Estimation of the bioavailability of phosphorus in cereals using a dynamic in vitro gastrointestinal model J. Sci. Food Agric., 74: 99-106).

Figure 4 shows the percentage of viable *S. cerevisiae* in the dynamic gastrointestinal model after digestion of a plain yoghourt supplemented with 1.6% of wild type or cell-lysis mutant yeast.

### Examples

### Example 1: Development of cell lysis yeast strains

### a) Saccharomyces cerevisiae

Lysis mutants (cly1 to cly8) (Table 1) described by Hartwell (Hartwell, L.H. (1967) Macromolecule synthesis in temperature sensitive mutants of yeast J.Bact., 93:, 662-1670) were obtained from Yeast Genetic Stock Centre (UCLA Berkeley) now integrated in ATCC (Rockville, Maryland, USA). These strains harbouring series of auxotrophic mutations were serially backcrossed to wild type strains of mating-type a (X2180-1A) or alpha (X2180-1B) (a gift from Thomas R.Manney), respectively in order to outcross all auxotrophic mutations.
Prototrophic segregants carrying the original temperature sensitive cly mutation were homozygously reconstituted to diploids with representatives carrying opposite mating types (Table 2).

Prototrophic segregants carrying the original temperature sensitive cly mutation were used to generate tetraploid strains according to the method described in patent EP 1 148 121, the disclosure of which is herewith incorporated herein by reference.

In order to combine cell lysis (Cly) phenotype and low temperature inactive (Lti) phenotype, diploidized prototrophic segregants being homozygous for mating type and cly mutation were crossed to diploid segregants as described in EP 1 148 121 carrying homozygously the low temperature inactive mutation and being homozygous for the opposite mating type. Such tetraploid zygotes were sporulated and diploid segregants exhibiting Cly phenotype and Lti phenotype were isolated and isolates carrying opposite mating types reconstituted to form tetraploids homozygously carrying cly and Iti mutations and thus exhibiting both (Cly and Lti) phenotypes. (Table 3)

**Table1: Haploid S.cerevsiae cell lysis strains:**

| original cell lysis mutants (ex. YGSC) : | |
|---|---|
| YGSC mutant | Genotype |
| 269 | a ade1 ade2 ura1 his7 lys2 tyr1 gal1 **cly1-1** |
| 315 | a ade1 ade2 ura1 his7 lys2 tyr1 gal1 ade9 **cly2-1** |
| X3124-4B | a arg4 lys7 trp1 gal1 **cly3-1** |
| 233 | a ade1 ade2 ura1 his7 lys2 tyr1 gal1 ade **cly4-1** |
| 254 | a ade1 ade2 ura1 his7 lys2 tyr1 gal1 **cly5-1** |
| 308 | a ade1 ade2 ura1 his7 lys2 tyr1 gal1 **cly6-1** |
| X3119-2B | a ura1 leu2 aro7 gal1 ade2 his2 lys **cly7-1** |
| 197 | a ade1 ade2 ura1 his7 lys2 tyr1 gal1 ade6 **cly8-1** |

**Table2: Diploid S. cerevisiae cell lysis strains:**

| | | |
|---|---|---|
| cly2: | | |
| CNCM I-4000 | a/alpha | cly2/cly2 |
| cly3: | | |
| CNCM I-4001 | a/alpha | cly3/cly3 |
| cly4: | | |
| CNCM I-4002 | a/alpha | cly4/cly4 |
| cly5: | | |
| CNCM I-4003 | a/alpha | cly5/cly5 |
| cly6: | | |
| CNCM I-4004 | a/alpha | cly6/cly6 |
| cly8: | | |
| CNCM I-4005 | a/alpha | cly8/cly8 |

**Table3: Tetraploid S. cerevisiae cell lysis strains:**

| | | |
|---|---|---|
| CNCM I-4006 | a/a/alpha/alpha | cly5/cly5/cly5/cly5 |
| CNCM I-4007 | a/a/alpha/alpha | cly5/cly5/cly5/cly5 lti/lti/lti/lti |

### b) Kluyveromyces marxianus

Mutagenesis: *Kluyveromyces marxianus* strain L46 (CNCM I-4008) (a natural isolate from the Nestlé strain collection) was pre-grown for 16 hours in 5 ml YPD (1% Difco Bacto Yeast Extract, 2% Difco Bacto Peptone, 2% Glucose) under agitation (200rpm) at 25°C. The cells were harvested by centrifugation and washed twice with sterile water. The cells were suspended in 5ml sterile water and the cell concentration was determined with the help of a counting chamber (Bürki-Türk chamber from Assistent (Sondheim, Germany). An aliquot containing 2x10⁸ cells was transferred to a microtube, the cells were sedimented by centrifugation and suspended in 1ml 100mM sodium phosphate buffer at pH 7. 30µl of ethyl methane sulfonate (Sigma-Aldrich Chemie GmbH, Buchs, Switzerland) cat.# M-0880) was added and the tube kept under agitation for 1 hour at 30°C.

The cells were again sedimented and washed once with 1ml water and twice with 1ml of 5% sodium thiosulfate pentahydrate (Fluka, Buchs, Switzerland) cat.# 72048. Surviving cells were spread on YPD plates (1% Difco Bacto Yeast Extract, 2% Difco Bacto Peptone, 2% Glucose solidified with 2% Difco Bacto Agar) and grown to colonies. These were replicated on new YPD plates, incubated at 37°C and temperature sensitive clones (showing no growth at 37°C) isolated for cell lysis assay described in Example 2 (Table 4).

**Table 4: Kluyveromyces marxianus wild-type and cell lysis strains**

| | |
|---|---|
| Original *Kluyveromyces marxianus* isolate: | L46 |
| Temperature sensitive (ts) mutants giving positive response in cell lysis assay were for example: | |
| CNCM I-4009 | |
| CNCM I-4010 | |
| CNCM I-4011 | |

### c) Schizosaccharomyces pombe

*S. pombe* wild type strain 968 (mating type h90) was a kind gift from P.Munz (Universität Bern, Berne, Switzerland); lysis strain Sp132 (mat h- cwg1-1 leu1-32) (Ribas, J.C et al. (1991) Isolation and Characterization of Schizosaccharomyces pombe. Mutants Defective in Cell Wall (1-3) β-D-Glucan, J.Bact. 173: 3456-3462) was kindly received from J.C. Ribas, Universidad de Salamanca, Salamanca, Spain.

### Example 2: Detection of cell lysis phenotype by adenylate kinase activity measurement

To assess cell lysis at non-permissive temperature we used the commercially available ToxiLight assay (Cambrex Bio Science, Rockland, ME USA) which quantitatively measures the intracellular Adenylate Kinase (AK) released in the supernatant by lysed cells.

Yeast strains were pre-cultured in 40ml YD medium (0.5% Difco Bacto Yeast Extract, 1.5% Glucose) up to an optical density (OD) at 600nm of approximately 0.5 - 4.0.

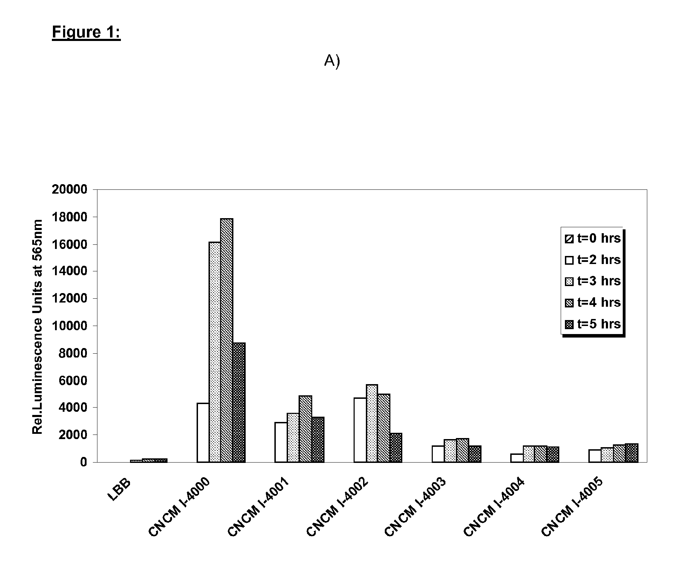

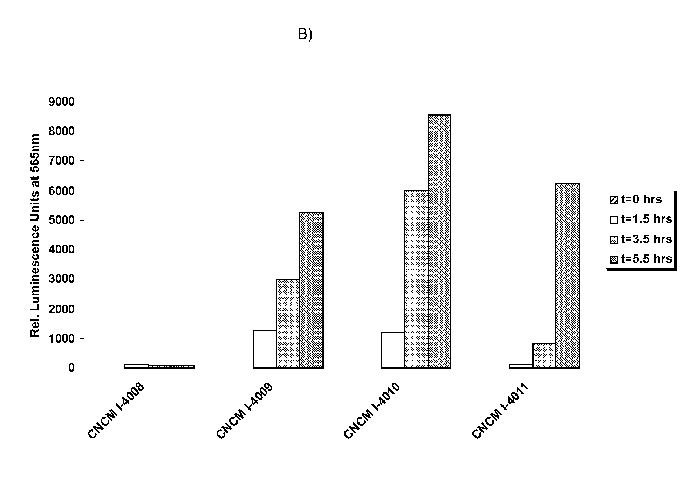

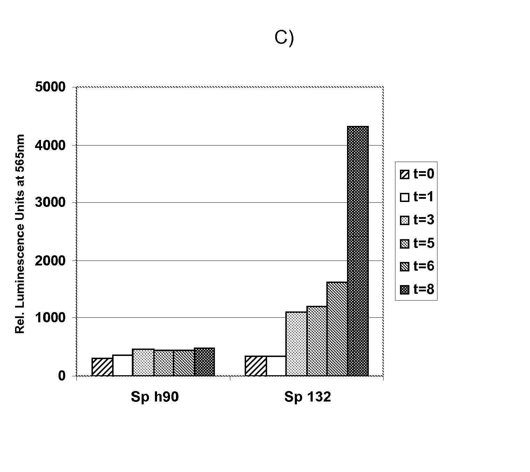

The cells were harvested by centrifugation and suspended in MV (0.67% Difco Yeast Nitrogen Base w/o amino acid, 2% Glucose, 2% Sodium succinate) to an OD at 600nm of 1.0. 20 ml of this suspension were incubated in 100ml erlenmeyers at 37°C under agitation (120 rpm). At desired time points 20µl of the cultures were transferred to a luminescence compatible 96 well plate and the luminescence, which is proportional to the amount of released AK, was read at 565 nm. AK release at 37°C from lysis and control strains is shown in Figures 1A - 1C. It is noteworthy that different mutations lead to different cell lysis speed, allowing targeted release of the compounds at different location along the digestive tract.

### Example 3: Iron accumulation in S. cerevisiae and K. marxianus under batch fermentation

Iron-enriched biomass was obtained by growing yeast in 1 L , 0.5% bacto yeast extract (Difco), 1.5 % glucose (Merck) and 5, 10, 15, 20 or 25 mM iron citrate (Sigma). After 3 days at 25°C the cells were harvested and washed 3 times in cold distilled water to remove non-incorporated iron salts. The iron load was determined by ICP-AES (Inductively coupled plasma atomic emission spectroscopy) on ashes. Ashes were obtained by destruction of the organic matter at 550°C during 8 hours in a muffle furnace. The values of incorporated iron resulting from growth with different iron citrate concentration in the medium are presented in table 5.

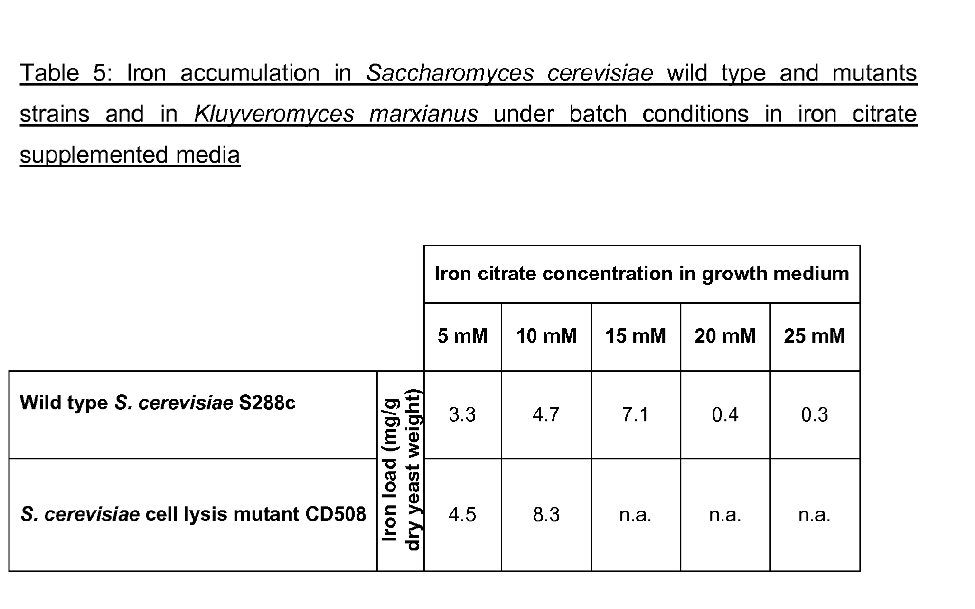

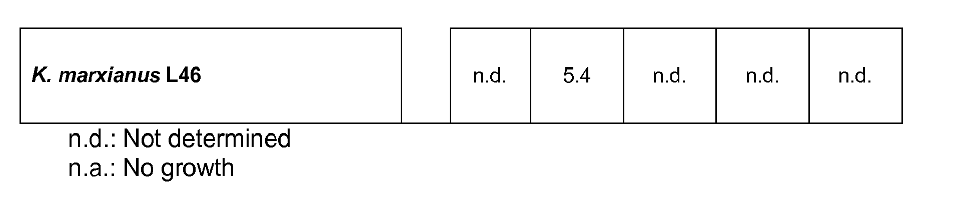

### Example 4: Cell-lysis and Iron release under non permissive conditions (37°C) by S. cerevisiae wild-type and mutant strains in gastric juice simulates

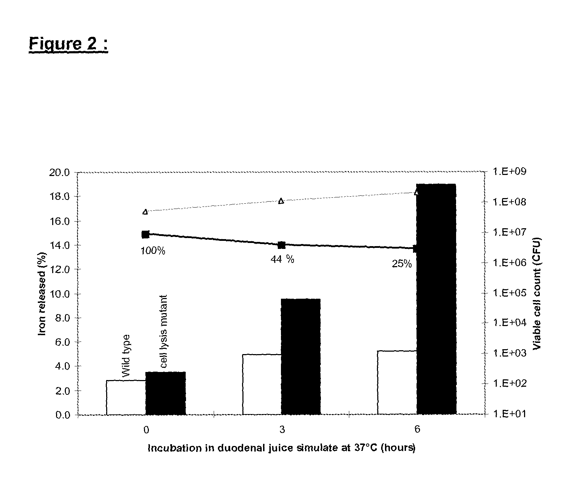

The iron release by yeast was determined in a gastric/duodenal juice simulate at 37°C in the presence of a carbon source to trigger cell lysis (Fig 2).

5 g of iron-loaded yeast biomass (water content 25 % w/w) were first placed in 80 ml gastric juice simulate containing 0.23% porcine pepsin (Sigma), 0.5% NaCl, 2 % glucose, pH2 during 30 min at 37°C under stirring. After 30 min yeast cells were pelleted by centrifugation, the supernatant was removed and used for iron release determination by ICP-AES.

On yeast pellet, 80 ml of a solution of duodenal juice simulate containing 0.27% pancreatin (Sigma), 0.54% bile extract (Sigma) and 2 % glucose in phosphate buffer 0.2 M pH 7 were added and the sample was further incubated under stirring at 37°C during 24 hours.

Sample aliquots were withdrawn after 0min, 30min, 1 h, 3h, 6h and 24 h to determine viable cell titer. The amount of iron released was measured in the supernatant after centrifugation and filtration on a 0.45 µm syringe filter.

The viable cell titer was determined after serial dilution of sample aliquots and plating on YPD plates (1% Difco Bacto Yeast Extract, 2% Difco Bacto Peptone, 2% Glucose solidified with 2% Difco Bacto Agar).

### Example 5: Digestion of yeast-supplemented yoghourt in the dynamic in vitro gastrointestinal model (TIM-1)

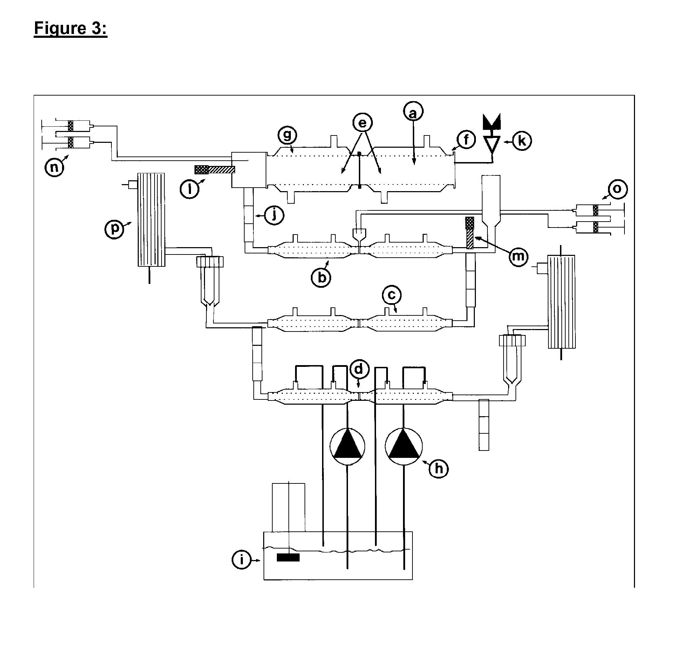

The model consists of four successive compartments (Fig 3) simulating the stomach, duodenum, jejunum and ileum. The gastrointestinal *in vitro* model has been described in detail (Minekus et al (1995) A multicompartmental computer-controlled model simulating the stomach and the small intestine. Alternative to laboratory animals (ATLA), 23: 197-209). Each compartment is formed by two connected basic units consisting of a glass jacket with a flexible wall inside. Water is pumped from a water bath into the glass jackets around the flexible walls to control the temperature inside the units and the pressure on the flexible walls. Changes in the water pressure are achieved by computer-activated rotary pumps. This enables mixing of the chyme by alternate compression and relaxation of the flexible walls. The compartments are connected by peristaltic valve pumps consisting of three connected T-tubes, each with a separate tube-like flexible wall inside. In the open position, the flexible walls facilitate the passage of the chyme, if pressure is applied to the outside of the flexible wall, the valve is closed. Peristaltic pumping is achieved by regulating the sequence of opening and closing of the three parts of the valve-pump. During each peristaltic cycle, a constant volume of chyme is transferred. The frequency of peristaltic cycles is dictated by a computer, allowing the flow rate of the chyme to be controlled. The volume in each compartment is monitored with a pressure sensor connected to the computer.

The gastric and duodenal compartments are equipped with pH electrodes. The pH values are controlled via the computer secreting either water or 1 M HCl into the stomach, or by secreting either water or 1 M NaHCO₃ into the duodenum via syringe pumps. Secretions of gastric electrolytes and enzymes, bile and pancreatic juices are regulated using computer controlled syringe pumps. The jejunal and ileal compartments are connected with hollow-fibre devices to absorb digestion products and water from the chyme and to modify electrolyte and bile salt concentrations in the chime.

The computer program has been designed to accept parameters and data obtained from *in vivo* studies in animals or human volunteers, such as the pH curves for the stomach and duodenum, secretion rates into the different compartments, water absorption from the small intestine and gastric and ileal delivery. To control the transit of the chyme, a power exponential formula for gastric and ileal delivery is used, as described by (Elashoff et al. (1982) Analysis of gastric juice emptying data. Gastroenterology, 83: 1306-1312).

At the start of the experiment the test material was introduced into the gastric compartment. The test meal was composed of 300g Hirz low fat plain yoghourt supplemented with 5.5% glucose and 1.6% wild-type or cell-lysis mutant yeast cells (dry weight).

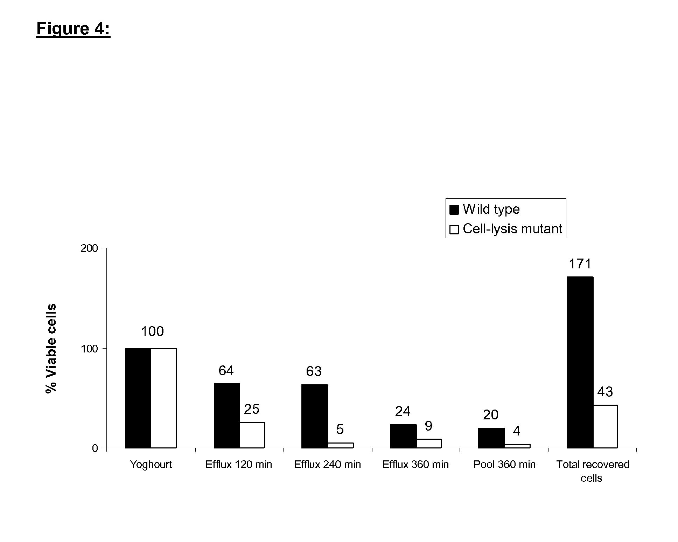

The stomach, duodenal and ileal juices were collected by syringe withdrawal directly in the compartments. The stomach and duodenal juices after 60 min, the jejunal and ileal juices after 2 and 4 hours. The ileal deliveries were collected after 2, 4 and 6 h in bottles. Jejunal and ileal dialysis fluids were collected after 2, 4 and 6 h and the volumes were measured. Results are shown in figure 4.

The wild type strain divided in the gastrointestinal systems at 37°C whereas the mutant lysed in the same conditions. Only 43% of the cells are recovered after 6 hours in the dynamic *in vitro* system.

### Example 6: Description of nutritional composition including iron-enriched yeast

**• Yogurt composition**

| | |
|---|---|
| | g per 100g |
| Milk fat | 3.730 |
| Milk | 89.010 |
| Skimmed milk powder | 1.980 |
| Starch | 0.240 |
| Sugar | 2.360 |
| Starter | 2.505 |
| Iron enriched yeast | 0.175 |

**• Fresh cheese white**

| | |
|---|---|
| | g per 100 g |
| Milk SNF | 14.1 530 |
| Sucrose | 7.4963 |
| Milk fat | 2.3858 |
| Vegetable fat | 1.7641 |
| Lactic cultures | 0.0019 |
| Vitamin D3 | 0.0003 |
| Water | 74.0236 |
| Iron enriched yeast | 0.1750 |

**• Infant formula**

| | | |
|---|---|---|
| | g per 100 g | |
| Protein equivalents | 1.40 | |
| Casein | | 0.6 |
| Whey protein | | 0.8 |
| Carbohydrates | 7.0 | |
| Lactose | | 1.3 |
| Glucose | | 0.2 |
| Maltose | | 2.1 |
| Polysaccharides | | 3.5 |
| Lipids | 3.6 | |
| Saturated | | 1.4 |
| Mono-unsaturated | | 1.7 |
| Poly-unsaturated | | 0.5 |
| Others | 0.1 | |
| Iron enriched yeast | 0.175 | |
| Water | 87.725 | |

## Claims

1. Use of a temperature sensitive micro-organism that will at least partially lyse in the temperature range of about 32 to 45°C as vehicle for the preparation of a composition to deliver compounds, in particular nutrients, to a portion of the body, in particular to the gastrointestinal tract.

2. Use in accordance with claim 1, wherein the micro-organism is yeast, for example a low temperature induced yeast, preferably a food grade yeast selected from the group consisting of hemiascetomycetous yeast species, *Ascomycotina* or *Deuteromycotina,* more preferably the yeast is selected from the group consisting of *Debaryomyces, Schizosaccharomyces, Kluyveromyces, Saccharomyces, Yarrowia, Zygosaccharomyces, Candida* and *Rhodotorula,* and preferably *Saccharomyces cerevisiae* (baker's yeast), most preferably, the temperature sensitive *S. cerevisiae* strains CNCM I-4000, CNCM I-4001, CNCM I-4002, CNCM I-4003, CNCM I-4004, CNCM I-4005, CNCM I-4006, CNCM I-4007, or the temperature sensitive *K. marxianus strains* CNCM I-4009, CNCM I-4010, CNCM I-4011; or bacteria, in particular selected from the group consisting of lactic acid bacteria, in particular *Lactobacilli* and/or *Bifidobacteria* and, more preferably, *Lactobacillus johnsonii, Lactobacillus reuteri, Lactobacillus rhamnosus, Lactobacillus paracasei, Lactobacillus casei, Bifidobacterium bifidum, Bifidobacterium breve, Bifidobacterium longum, Bifidobacterium animais, Bifidobacterium lactis, Bifidobacterium infantis, Bifidobacterium adolescentis, Bifidobacterium pseudocatenulatum, Bifidobacterium catenulatum* or mixtures thereof and most preferably a temperature sensitive bacterium derived from the strains *Lactobacillus johnsonii* NCC 533 (CNCM I-1225), *Lactobacillus paracasei* NCC2461 (CNCM I-2116), *Lactobacillus rhamnosus* NCC 4007 (CGMCC 1.3724), *Bifidobacterium adolescentis* NCC 251 (CNCM I-2168), *Bifidobacterium longum* NCC 490 (CNCM I-2170), NCC 2705 (CNCM I-2618) and NCC 3001 (ATCC BAA-999), *Bifidobacterium lactis* NCC 2818 (CNCM I-3446), *Bifidobacterium breve* NCC 2950 (CNCM I-3865), NCC 466 (CNCM I-3914) or mixtures thereof.

3. Use in accordance with one of the preceding claims, wherein the temperature sensitive micro-organism is obtained by natural selection.

4. Use in accordance with one of the preceding claims, wherein the yeast is temperature sensitive due to a mutation in the pkc1 gene.

5. Use in accordance with one of the preceding claims, wherein the temperature sensitive *S. cerevisiae* yeast is selected among mutant strains consisting of genetic complementation groups cly 1, cly 2, cly 3, cly 4, cly 5, cly 6, cly 7, cly 8 or combinations thereof.

6. Use in accordance with one of the preceding claims, wherein the yeast is *S. cerevisiae* and temperature sensitive due to an alteration in the genes regulating the polarized growth, the septation, and/or the maintenance of the cell wall integrity, wherein the genes are preferably selected from the group consisting of *BCK1, CDC12, CDC15, CDC31, CDC24, GLC7, GPI7, HSF1, IQG1, KIC1, LST8, MID2, WSC1, MOT2, MPK1, MPT5, SSD1, MSS4, NUD1, PKC1, PMT2, PMT4, PPH22, PPZ1, PPZ2, RH03* and *RHO4, ROM1, ROM2, SRB1, SWI4, SWM1, TIF51A, TSC11, TOR1, TCO89, TAP42, TOR2, BIG1, TUS1, VIG9, WSC1, WSC2, WSC3, YPK1, YPK2, YPK1, YKR2,* the seven yapsins, and combinations thereof, and/or wherein the yeast is *S. pombe* and temperature sensitive due to an alteration in the genes regulating the polarized growth, the septation, and the maintenance of the cell wall integrity, wherein the genes are preferably selected from the group consisting of *ags1, bgs3, bgs4, cwg1, orb11, cps1, cwg1, cwg2, ehs1, PP2C, psu1, rgf1, rgf3, rho1, sid1, sid2, sid4, spg1* and *mob1,* and combinations thereof

7. Use in accordance with one of the preceding claims, wherein the nutrients are selected from the group consisting of minerals, preferably iron, selenium, zinc, chromium, copper; vitamins, preferably folic acid, niacin, vitamin C; plant polyphenols, preferably catechins, chlorogenic acids, isoflavones; carotenoids, preferably beta-carotene, astaxanthin, zeaxanthin, lycopen; alkaloids , preferably caffeine; amino acids , preferably cysteine, essential amino acids such as isoleucine, leucine, lysine, threonine, tryptophan, methionine, histidine, valine and phenylalanine; antioxidants; aroma compounds , preferably lemon oil, orange peel oil, thyme, rosemary oil, oregano oil; lipids, preferably polyunsaturated fatty acids, long-chain polyunsaturated fatty acids, fatty amides, glycerophospholipids, glycerides; enzymes and/or co-enzymes, preferably co-enzyme Q10, beta-galactosidase; non secreted heterologous proteins, preferably vaccines or mixtures thereof.

8. Use in accordance with one of the preceding claims, wherein the composition is a food composition, a dietary supplement, a nutraceutical, a drink, an animal food composition, a cosmetic composition, or a medical composition.

9. Use in accordance with claim 8, wherein the food composition is a cereal product, such as a cereal bar, baby cereals and infant cereals or a chilled dairy product such as a cheese, milk, fermented milk drink, milk based fermented products, curd, ice cream, yoghurt, milk powder and fortified powder drinks such as infant formula and growing-up milks, beverages, sport nutrition and drink formulation, soups, culinary products, pet food, tablets, liquid bacterial suspensions, dried oral supplements, wet oral supplements, compositions for dry tube feeding and/or compositions for wet tube feeding.

10. Use in accordance with one of the preceding claims wherein the compounds, in particular nutrients, are incorporated into the yeast by growing the yeast on a medium enriched with these compounds and/or by the yeast synthesising the compounds itself.

11. Use in accordance with one of the preceding claims to improve the stability of compounds, in particular nutrients, and/or to reduce a detrimental impact of the compounds, in particular nutrients, on taste.

12. Use in accordance with one of the preceding claims to limit the interactions of the compounds, in particular nutrients, with other compounds.

13. Use in accordance with one of the preceding claims to enable a targeted release of compounds, in particular nutrients, preferably to the gut or a specific location in the digestive tract.

14. Use in accordance with one of the preceding claims to improve compound, in particular nutrient, bioaccessibility and/or bioavailability.

15. A yeast strain selected from the group consisting of CNCM I-4000, CNCM I-4001, CNCM I-4002, CNCM I-4003, CNCM I-4004, CNCM I-4005, CNCM I-4006, CNCM I-4007, CNCM I-4009, CNCM I-4010, CNCM I-4011.
